# EUROPEAN PATENT SPECIFICATION

(11) **EP 0 386 218 B1**
(45) Date of publication and mention of the grant of the patent: **10.01.1996**
(21) Application number: 89910276.8
(22) Date of filing: 24.08.1989
(51) Int. Cl.: A61B 5/00

(54) **REMOTE SENSING TONOMETRIC CATHETER APPARATUS AND METHOD**
TONOMETRISCHE KATHETERVORRICHTUNG UND VERFAHREN MIT FERNSENSOR
PROCEDE ET APPAREIL A CATHETER TONOMETRIQUE DE DETECTION A DISTANCE

(30) Priority: 26.08.1988 US 237287; 13.07.1989 US 380706
(43) Date of publication of application: 12.09.1990
(73) Proprietor: MOUNTPELIER INVESTMENTS, S.A., FL-9490 Vaduz (LI)
(72) Inventor: FIDDIAN-GREEN, Richard, G., Boston, MA 02116 (US)
(74) Representative: Spall, Christopher John
(86) International application number: US8903659
(87) International publication number: WO9001893

(56) References cited:
- EP-A- 0 149 866
- US-A- 3 480 003
- US-A- 4 020 830
- US-A- 4 176 659
- US-A- 4 265 249
- US-A- 4 273 636
- US-A- 4 338 174
- US-A- 4 381 001
- US-A- 4 432 366
- US-A- 4 534 825
- US-A- 4 643 192
- US-A- 4 727 730
- Annals of Biomedical Engineering, Vol. 11, pages 499-510, 1983; Vurek et al.:" A fibre optic probe pCO2 sensor"

## Description

### Background and Summary of the Invention

This invention relates to medical diagnostic equipment and methods and is particularly concerned with hollow viscus tonometry and remote electronic and optical sensing.

The prior art (see U.S. Patent No. 4,643,192) has recognized that intestinal ischemia, and to a lesser degree, stress ulceration, are two problems that plague physicians involved in the management of patients in intensive care units. Intestinal ischemia, in particular, has an insidious onset and may not be detected until days after the intestine has become completely and irreversibly compromised. A delay in the diagnosis of intestinal ischemia may have devastating consequences for a patient. The availability of means for early diagnosis and management of patients with these problems would have immediate applicability in all intensive care units, especially where the procedure can be conveniently conducted with reasonable safety and reliability.

It has been established that a fall in the intramucosal pH may precede the development of intestinal ischemia and stress ulceration. As I reported in my prior U.S. Patent No. 4,643,192, expressly incorporated herein by reference, entitled "Hollow Viscus Tonometry" a fall in intramucosal pH also occurs within minutes of inducing intestinal ischemia in dogs. The fall in pH in intestinal mucosa, and hence the likelihood of ischemia or stress ulceration, can be reliably calculated from a pCO₂ (partial pressure of CO₂), or other indicia of pH, in luminal fluid and the bicarbonate concentration in arterial blood. The method of calculating the pH in intestinal mucosal tissue, pursuant to principles of my prior patent, has been validated by directed measurements under a variety of conditions simulating clinical problems. A correlation coefficient in the order of 0.92 to 0.95 has been obtained in each of 16 dogs. The validity of the procedure is inherently extensible to humans, and indeed may also be useful in assessing the vitality of other hollow organs and tissue. See R.G. Fiddian-Green et al. "Splanchnic Ischemia and Multiple Organ Failure".

To measure the pCO₂ in the lumen of the gut it has heretofore been necessary to obtain and remove a sample of fluid that has been in contact with the wall of the gut for a certain time period, usually at least half an hour. It has now been observed that it is somewhat difficult to manually aspirate the sampling fluid or medium from a tonometric catheter located in the gut or other internal focus with any consistency. It is much easier to obtain such samples from the stomach, but samples obtained from the stomach frequently contain foreign material that can damage a gas analyzer.

As taught in my prior patent, the desired sample or samples can be obtained from the gut using a catheter tube (called a tonometric catheter) having a walled sampling chamber on the tube with the sampling chamber being in sample-specific communication with the hollow interior of the tube. The wall of the sampling chamber comprises a material which is substantially impermeable to liquid yet is highly permeable to gas. One suitable material is polydimethylsiloxane elastomer.

In use the catheter is introduced into a patient to place the sampling chamber at a desired site within the gut. An aspirating liquid or medium is employed to fill the interior of the sampling chamber. The sampling chamber is left in place at the desired sampling site long enough to allow the gases present to diffuse through the wall of the sampling chamber into the aspirating liquid. The time should be long enough for the gases to equilibrate. The liquid impermeable nature of the sample chamber wall material prevents both the aspirating liquid from leaking out of the chamber and also the intrusion of any liquids into the aspirating liquid. After the appropriate or desired amount of placement time has elapsed the aspirating liquid is aspirated along with the gases which have diffused into it. The sample thus obtained is analyzed for gas content, in particular for pCO₂. In this way the pCO₂ within the lumen of the gut can be reliably measured with the fluid being free from lumenal debris.

In carrying out the diagnostic method taught in my prior patent the pCO₂ measurement is utilized in conjunction with a measurement of the bicarbonate ion concentration (HCO₃⁻) in an arterial blood sample of the patient for determining the pH of the tract wall.

Depending upon the particular condition of a given patient, the catheter may be left in place and samples may be taken at periodic intervals so that pH values may be periodically calculated. The procedure has a high reliability in accurately determining the adequacy of organ tissue oxygenation, and diagnosing intestinal ischemia in its incipient stages. Such determination or detection can be useful in treating the patient so that the potentially devastating consequences resulting from less timely detection may often be avoided.

While the sampling techniques taught in my prior patent have provided highly accurate and reliable results, it has now been observed that there are instances (in the care of the critically ill in intensive care units, for example) in which remote sensing of the organ or organ-wall condition and automatic calculation of the organ or organ-wall pH would be advantageous and easier to effectuate. This method would thus partially or totally eliminate the need for the somewhat cumbersome aspiration of the sampling fluid or medium which fills the sampling chamber; it may also eliminate the need for the sampling chamber to be in sampling-medium communication with any other part of the device. There is also a need to extend the benefits of tonometric sampling and sensing to other internal hollow viscous organs. To this end, there is a need for new and different tonometric devices specifically adapted to allow my sensing and sampling techniques to be performed with ease in a clinical environment, and in combination with other procedures.

The importance and significance of determining the pH of the wall of a given hollow viscous organ has been recently dramatically magnified as a result of the recent recognition that the pH of the wall of a given organ can be employed to accurately evaluate the vitality and/or stability of that organ as well as others; this is in contrast to merely determining whether such an organ is experiencing an ischemic event. Further, certain organs can be selected for monitoring, either alone or in combination, and evaluation of this organ or these organs can aid in predicting the overall condition of the patient, or the onset of a multitude of pathologies, including predicting or identifying such events as multiple organ failure. Such a methodology can be employed to greatly enhance and supplement the monitoring of the critically ill, for example.

According to the present invention, a tonometric catheter apparatus for remotely determining a fluid or gas property indicative of the condition of a hollow internal organ of a human or other mammal in vivo as defined by said fluid or gas property is provided, comprising:
(a) an elongated tonometric catheter tube having a lumen extending longitudinally therethrough, said tube being composed of a first elastomeric material that is substantially impermeable to one or more fluids or gases of interest, said fluids of interest including oxygen gases and carbon dioxide gases;
(b) at least one walled sampling chamber on said catheter tube in fluid communication with the interior of said lumen, said walled sampling chamber being defined by a balloon member generally surrounding a portion of said tube and sealingly interconnected therewith, said balloon member being adapted to form an interior space between said balloon member and said catheter tube, the wall of said balloon member being composed of a second material that is freely and selectively permeable to said one or more fluids or gases of interest, said second material being substantially impermeable to other fluids or gases, with said tube extending to a position outside of the body of the human or other mammal, and with said lumen providing fluid communication between said sampling chamber and the outside of the body of the human or other mammal;
(c) sensor means for sensing the level of at least one of said fluids or gases of interest present within said sampling chamber;
characterised in that: said sensor means is disposed within said sampling chamber; said apparatus further comprises calibration means for introducing a calibration fluid into said sampling chamber through said lumen in order to calibrate or recalibrate said sensor means; and a portion of said balloon member is selectively positioned substantially adjacent to a wall portion of the internal organ in order to allow said one or more fluids or gases of interest from the tissue of the wall portion of the internal organ to permeate into said sampling chamber, the sensor means being adapted to sense the level of at least one of the fluids or gases of interest from the tissue of the wall portion of the internal organ, and the calibration means being operable whilst said balloon member is substantially adjacent to the wall portion of the internal organ.

The invention may comprise an apparatus for determining the pH of a hollow internal organ of a human or other mammal, characterised in that: said tube is composed of a first elastomeric material that is substantially impermeable to a fluid or gas indicative of pH; said wall of said balloon member is composed of a second material that is freely and selectively permeable to said fluid or gas indicative of pH; a non-temperature sensor is disposed on said tonometric catheter tube in communication with said sampling chamber for introduction into said organ with said tonometric catheter, said sensor comprising means responsive to a fluid or gas property indicative of pH and means for developing an electromagnetic signal indicative of said fluid or gas property; a means is provided responsive to said signal for generating a pH signal indicative of the pH of said organ; and said position of said balloon member substantially adjacent to said wall portion of said hollow internal organ is such to allow said fluid or gas indicative of pH from the tissue of the wall portion of the internal organ to permeate into said sampling chamber.

The invention may comprise an apparatus for determining the condition of a hollow internal organ of a human or other mammal as defined by the fluid pressure and the blood pH indicative of the condition of the hollow internal organ, characterised in that: said tube is composed of a first elastomeric material that is substantially impermeable to a fluid or gas indicative of the fluid pressure of the hollow internal organ; said wall of said balloon member is composed of a second material that is freely and selectively permeable to said fluid or gas indicative of the fluid pressure of the hollow internal organ; a non-temperature sensor is disposed on said tonometric catheter tube in communication with said sampling chamber for developing a first electromagnetic signal indicative of said fluid pressure condition of said hollow internal organ; a means is provided for developing a second electromagnetic signal indicative of said blood pH of said hollow internal organ; a means is provided receptive of said first and second signals for generating a third signal indicative of the condition of said hollow internal organ; a means is provided responsive to said third signal and located outside said organ for reporting said condition of said hollow internal organ; and said position of said balloon member substantially adjacent to a wall portion of said hollow internal organ is such to allow said fluid or gas indicative of the fluid pressure from the tissue of the wall portion of the internal organ to permeate into said sampling chamber.

In one embodiment, a chemically sensitive electronic transducer (or plurality of transducers), such as a field effect transistor, is attached to a tonometric catheter for introduction into the organ along with the tonometric catheter. The first electronic sensor, preferably non-temperature, generates and conveys an electromagnetic signal indicative of some desired aspect of organ condition, e.g., indicative of the pCO₂, pH and/or pO₂ level of the organ or organ-wall. For example, in one preferred embodiment, mean ambient pCO₂, pH and/or pO₂ of lumenal fluid or the like is measured or monitored via wire or other suitable electromagnetic energy conveying means to an electronic circuit which interprets the electromagnetic signal and produces a report of the organ condition. The electronic circuit may include an input for receiving a separately determined signal indicative of the blood pH of the patient. Using this pCO₂, pH and/or pO₂ measurement along with blood (preferably arterial) pH data, the electronic circuit determines the pH of the organ wall under test and thereby provides information for determining the organ's current condition or perhaps predicting the organ's future condition. The electronic circuit may be suitably constructed from analog components, digital components or both.

In another embodiment, a pH, pCO₂ or pO₂ sensitive colorimetric substance is injected into an area adjacent to the organ, e.g., into the sampling chamber of the tonometric catheter, and an optical sensor is employed to detect color change in order to determine the pH of the wall of that organ. The optical sensor can either be disposed in or on the tonometric catheter for introduction into the area adjacent the organ or it may be disposed outside the organ with fiber optic cable optically coupling the sensor to the tonometric catheter site at which the pH sensitive substance has been injected.

In another form the invention may provide a variety of tonometric catheter devices for sensing and/or sampling a fluid or gas property (such as pH, pO₂, pCO₂, and the like) which is indicative of the condition of an internal organ, in conjunction or combination with a walled catheter tube adapted for delivery or draining fluids, such as nasogastric tubes, urinary catheters, ureteric catheters, intestinal feeding tubes, wound or abdominal drains (suction or regular) and biliary tubes, catheters and stents, with or without remote sensing means for pH, pCO₂ and/or pO₂.

In still another form, the device may employ two separate walled catheter tubes, one tonometric catheter tube for the measurement of a fluid or gas property, that is in communication with the sampling chamber; and a second walled catheter tube adapted for delivering or draining fluids.

In yet another form, the device may employ a walled sampling chamber in communication with a sensing means, and a second walled catheter tube adapted for delivering or draining fluids.

Optionally, when a non-temperature sensing-means is employed, a second sensing-means may be employed as well.

For a more complete understanding of the invention, its objects and advantages, reference may be had to the following specification and to the accompanying drawings.

### Brief Description of the Drawings

Figure 1 is a plan view of a first embodiment of the tonometric catheter;
Figure 2A is a partial cross-sectional view of the tonometric catheter illustrating a first means for attachment of an electronic field effect transistor sensor;
Figure 2B is a partial cross-sectional view of the tonometric catheter illustrating a second means of attachment of the field effect transistor sensor;

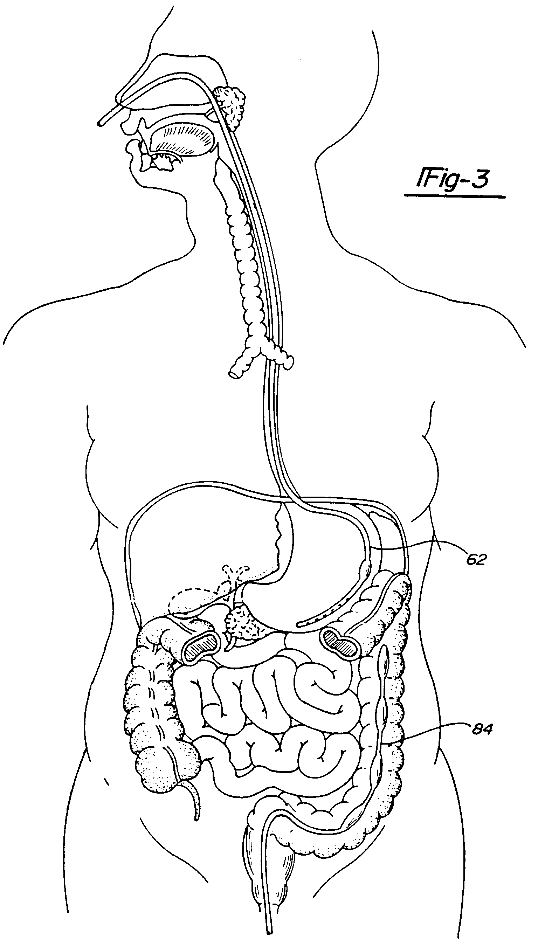

Figure 3 illustrates the method of use of the tonometric catheter in measurement of the pH of the colon and also of the stomach, the specific embodiment illustrated for colonic measurement being that of Figure 5 and the specific tonometric catheter for gastric measurement being that of Figure 4;
Figure 4 is another embodiment of the tonometric catheter with nasogastric tube;
Figure 4A is a cross-sectional view of the tonometric catheter of Figure 4 taken substantially along the line 4A-4A of Figure 4;
Figure 4B is a cross-sectional view of the tonometric catheter of Figure 4 taken substantially along the line 4B-4B of Figure 4;
Figure 5 is yet another embodiment of the tonometric catheter having multiple sensing/sampling portions;
Figure 5A is a cross-sectional view of the tonometric catheter of Figure 5, taken substantially along the line 5A-5A of Figure 5;

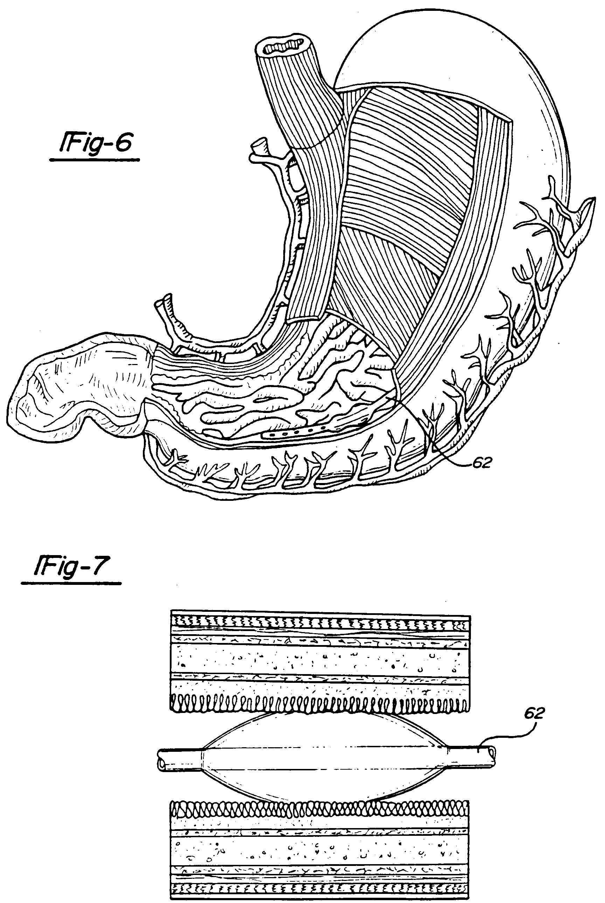

Figure 6 is a detailed view illustrating the tonometric catheter of Figure 4 in use within the stomach;
Figure 7 is a detailed view illustrating the tonometric catheter of Figure 5 in use within the colon;

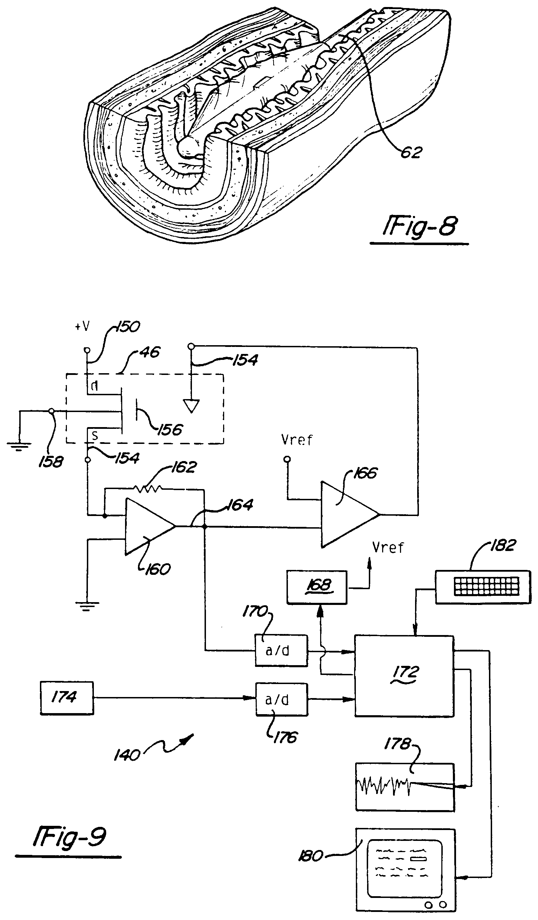

Figure 8 is a similar view illustrating the tonometric catheter of Figure 1 in use within the colon;
Figure 9 is an electrical schematic diagram illustrating one embodiment of electronic circuit in accordance with the invention;
Figure 10 is an electrical schematic diagram illustrating another embodiment of the optical measurement of pH in accordance with the invention;
Figure 11 is another embodiment of a tonometric catheter with a urinary catheter;
Figure 11A is a cross-sectional view of the tonometric catheter/urinary catheter of Figure 11, taken substantially along the line 11A-11A of Figure 11.

### Description of the Preferred Embodiments

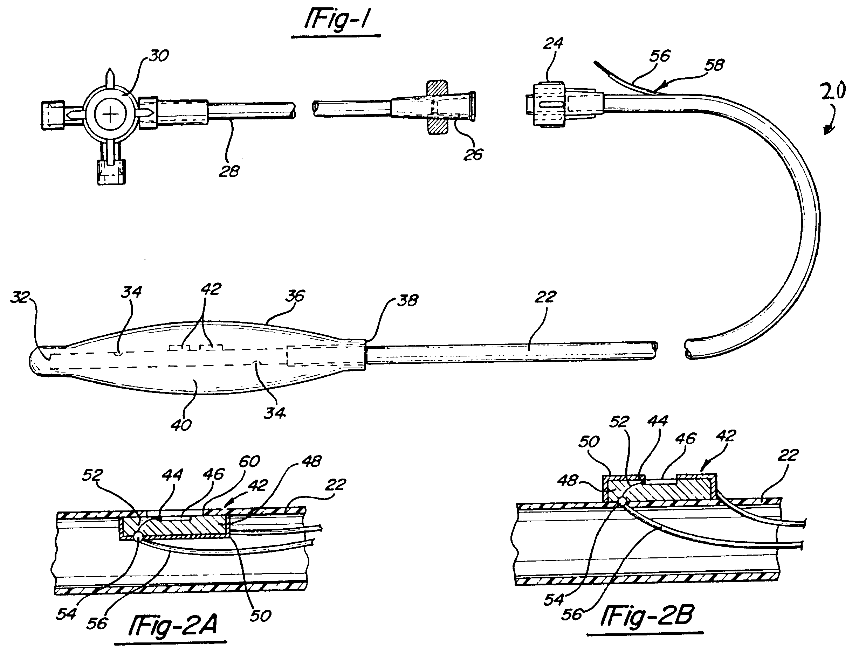

Figure 1 illustrates a first embodiment of tonometric catheter 20. The tonometric catheter comprises a length of suitable tubing 22, one end 32 of which is closed, and the opposite end of which has a connector such as a luer-lock 24. Luer-lock 24 is adapted to receive a complementary fitting 26, which in turn couples through a second length of tubing 28 to a three-way stopcock 30. Three-way stopcock 30 may be used to selectively connect tubing 28 to various sources of irrigation or aspiration.

Adjacent the closed end 32, tubing 22 is perforated as at 34. A balloon-like tonometric catheter membrane 36 is fitted over the closed end so that the perforations 34 are enclosed, as illustrated. The tonometric catheter membrane 36 has an internal sleeve diameter at 38 which forms a tight fit with tubing 22. The preferred form of tonometric catheter membrane is polydimethylsiloxane elastomer. The membrane may be sealed to the tubing 22 with appropriate adhesive so that the tonometric catheter membrane is sealed in a closed relationship to the outer wall of tubing 22, thereby forming a sampling chamber 40 adjacent closed end 32. The tonometric catheter membrane has a certain elasticity to allow the membrane to expand when filled with an aspirating liquid in order to contact the wall of the organ under examination, as will be explained below.

The membrane 36 is preferably constructed such that at least a portion of it is selectively permeable to the gas or fluid property of interest. In a preferred embodiment, it is selectively permeable to hydrogen, oxygen, or H⁺, so that pH, pCO₂ and/or pO₂ can be measured. It is also preferably impermeable to other materials that would interfere with the desired measurements, such as other gases, proteins, and the like. In a highly preferred embodiment, an ion-selective membrane is employed.

Bonded to either the inner wall or the outer wall of tubing 22 are one or more sensors 42 for detecting a property indicative of pH and/or temperature. Two such sensors are illustrated in Figure 1, bonded to the outside wall of tubing 22 with suitable adhesive. Figures 2A and 2B illustrate two alternate means of sensor attachment, Figure 2A illustrating the sensor attached to the inner wall of tubing 22 and Figure 2B illustrating the sensor attached to the outer wall of tubing 22.

In a preferred embodiment, at least a portion of the tubing, but not all of it, is made of a CO₂ impermeable material, such as polyester elastomers derived from the reaction of dimethylterephtalate 1,4-butanediol and α-hydro-Ω-hydroxypoly (oxytetramethylene). In a highly preferred embodiment, this is a material such as Hytril, sold by DuPont.

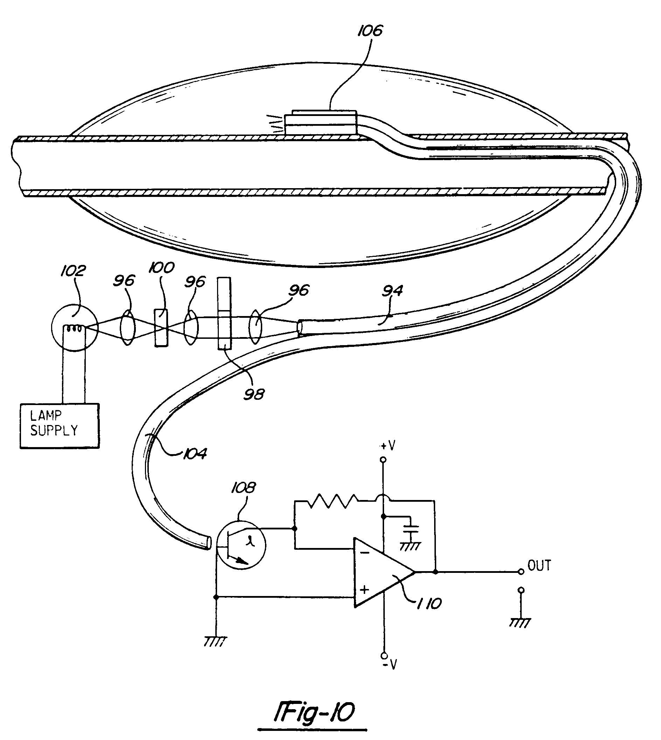

For purposes of sensing temperature, thermistor devices are presently preferred. For sensing properties indicative of pH chemically responsive field effect transistors or "Chemfets" may be employed. In this regard, Chemfet sensors 44 have been illustrated in Figures 2A and 2B. Chemfet sensor 44 comprises a field effect semiconductor device 46, which is encapsulated in a solution impervious material 48, such as a polymerized epoxy resin. The encapsulation material 48 in turn may be encapsulated in a housing 50 (Figure 2A). Semiconductor device 46 is electrically coupled by bonding wires 52 to a terminal 54. Suitable electrical conductors such as conductor 56 are attached to terminal 54 for electrically communicating between the Chemfet device 44 and the electronic circuitry described below in connection with Figure 9. Conductor 56 is preferably routed through tubing 22 and exits through a sealed aperture at or near the luer-lock end of tubing 22, as at 58. A more detailed description of a suitable electronic sensor may be found in U.S. Patent No. 4,020,830 to Johnson, entitled "Selective Chemical Sensitive FET Transducers," incorporated herein by reference. In order to allow a solution to contact the chemically sensitive surface of semiconductor device 46, tubing 22 may be provided with an aperture 60 when implementing the embodiment of Figure 2A. Such an aperture is not needed in the embodiment of Figure 2B, since the semiconductor device 46 is exposed to sampling chamber 40 by virtue of the external mounting configuration.

The sampling chamber 40 can be filled with an aspiration or sampling medium that is used to absorb or otherwise provide a means for incorporating and delivering or measuring the the fluids or gases of interest. Such a medium is selected depending upon many factors, including the properties of the fluids or gases of interest, the type of sensor 42 employed, and the type of calibration that is necessary. Such mediums include bicarbonate solutions and saline solution. It might be noted that gases often behave as fluids and are therefore frequently considered to be fluids.

As noted above, when the sensor employed does not require frequent recalibration, the need for the sampling chamber 40 to be in communication with the proximate end of the tonometric catheter (that remains outside the patient) may be eliminated since no aspiration is needed. However, in many instances such communication may still be desirable as aspiration may be required to calibrate the sensor or sensors, to replace the aspirating or sampling medium with a fresh medium, and to incorporate the gas or gases of interest.

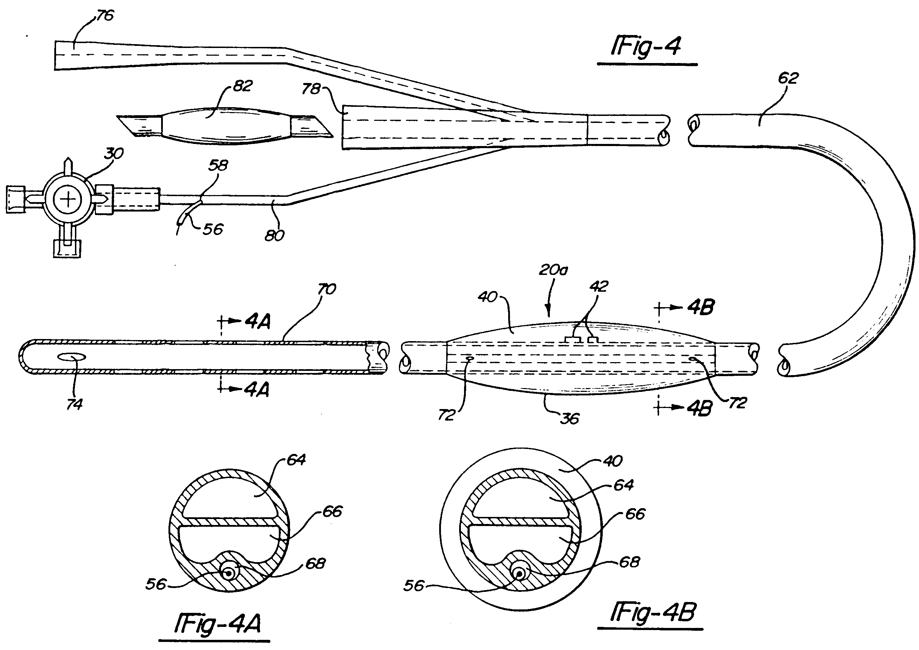

Another embodiment of the tonometric catheter is illustrated in Figures 4, 4A and 4B. As illustrated, the tonometric catheter is appropriately configured to also serve as a nasogastric sump, either with or without gastric suction. With reference to Figure 4, the tonometric catheter 20a comprises a multipassage tubing 62 which defines three individual noncommunicating (between each other) passageways or lumens, an air lumen 64, an optional suction lumen 66 and a tonometric catheter lumen 68. A tonometric catheter membrane, similar to that previously described, is attached at an intermediate location on tubing 62, allowing a portion of the tubing to extend beyond the end of membrane 36 to define the nasogastric sump 70. Tubing 62 is provided with a plurality of perforations 72 which communicate between tonometric catheter lumen 68 and the sampling chamber 40 defined by membrane 36. If desired, one or more sensors 42 can be included in accordance with the above teachings, in which case a suitable conductor 56 may be routed through tonometric catheter lumen 68 to exit at sealed aperture 58.

The nasogastric sump portion 70 is suitably provided with a plurality of openings 74 through which the stomach may be aspirated.

At the opposite end of tubing 62 the tubing splits to form three separate connections. Air lumen 64 communicates with air lumen passageway 76, suction lumen connects with suction lumen passageway 78 and tonometric catheter lumen 68 communicates with tonometric catheter lumen passageway 80. The tonometric catheter lumen passageway is fitted with three-way stopcock 30, similar in function and purpose to the three-way stopcock 30 described in connection with Figure 1. If desired, a quick connect fitting 82 may be used to couple the suction lumen passageway 78 with an aspiration source. As illustrated, the quick connect fitting preferably has angularly cut ends and a slightly enlarged midsection, making it easy to insert into the end of passageway 78 and also into the aspiration hose coupling (not shown). The enlarged midsection helps form a seal with the adjoining passageways. Preferably the quick connect fitting is fabricated of disposable plastic.

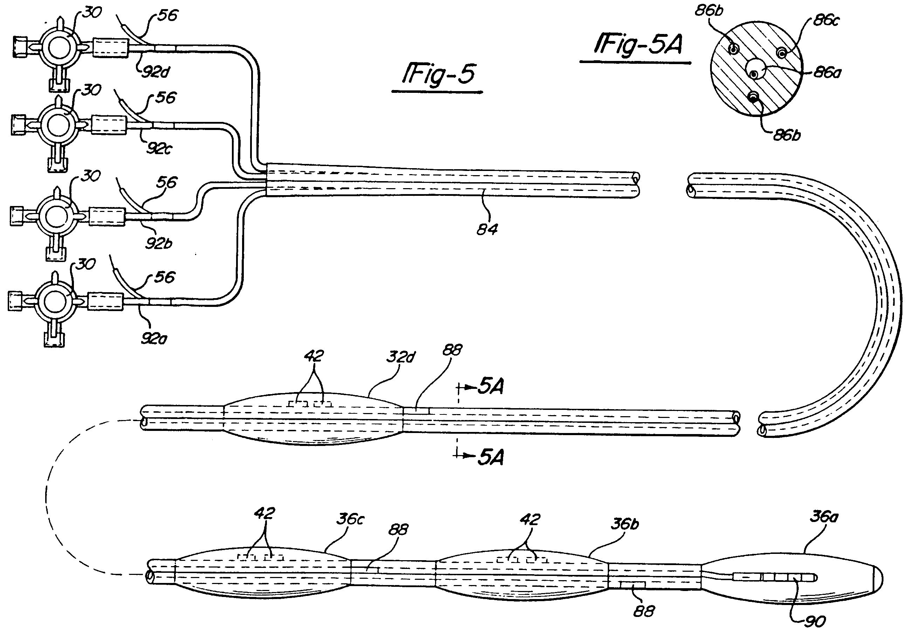

Yet another embodiment of the tonometric catheter is illustrated in Figures 5 and 5A. This embodiment is a multiple tonometric catheter embodiment employing a tubing 84 having a plurality of passageways or lumen as shown in the cross-sectional view of Figure 5A. Specifically, tubing 84 includes an air lumen 86a which communicates with the endmost tonometric catheter 36a and three additional tonometric catheter lumens 86b, 86c and 86d, which communicate respectively with tonometric catheters 36b, 36c and 36d. As with the other embodiments, each tonometric catheter may be provided with one or more sensors such as sensors 42. A radiopaque tungsten plug 88 is positioned within each of the three tonometric catheter lumen 86b, 86c and 86d adjacent the distal end of each tonometric catheter, serving to block the remainder of the tonometric catheter lumen passageway and thereby ensuring that fluid pressure introduced into each tonometric catheter lumen will cause the associated tonometric catheter to balloon outwardly as required during use. Similarly, a radiopaque tungsten rod 90 is fitted as a plug in the end of air lumen 86a, serving to terminate the end of the air lumen passageway. Being radiopaque, the tungsten plugs and tungsten rod aid in properly positioning the tonometric catheters by being visible under fluoroscope or x-ray. In addition, if desired, tubing 84 can be provided with a radiopaque stripe along all or part of its length.

At the proximal end of tubing 84 the lumen 86a-86d diverge to define four separate tubes 92a-92d. Each tube is fitted with a three-way stopcock similar to those described above. Each sampling connector may optionally be coded numerically by color, etc. While four approximately equally spaced tonometric catheters have been illustrated in Figure 5, it will be understood that the invention can be modified to include a greater or fewer number of tonometric catheters at different spacing as required for a particular application. It will also be understood that some or all of the tonometric catheters can include one or more sensors coupled to conductors 56, each preferably routed through the corresponding lumen passageway.

Referring now to Figure 9, a suitable electronic monitoring circuit will now be described. In Figure 9 CHEMFET semiconductor device 46 has been shown schematically by the equivalent circuit model enclosed in dotted lines. The device 46 thus comprises drain electrode 150, source electrode 152 and reference electrode 154. The chemically selective system, such as a membrane system is depicted diagrammatically at 156. The substrate is grounded as at 158.

Source electrode 154 is coupled to an input lead of operational amplifier 160 which includes feedback network diagrammatically depicted at 162. Operational amplifier 160 senses the drain source current flowing through device 46 and converts this signal into a voltage signal which is output on lead 164. The drain source current changes in accordance with changes in the chemical system under test. More specifically, as the pCO₂ level changes in the fluid exposed to device 46, the drain source current changes accordingly. Hence the output voltage signal on lead 164 is likewise an indication of the pCO₂ level of the organ under test. This voltage signal on lead 164 is coupled to an input of comparator 166 which also receives a reference voltage V_{ref}, which may be supplied using a voltage divider network (not shown) or which may alternatively be provided by a digitally controlled voltage source 168. The output of comparator 166 is fed to reference electrode 154 to provide a stable reference bias voltage. If a digitally controlled voltage source is used, this reference voltage can be adjusted and calibrated by a computer circuit yet to be discussed. The voltage signal on lead 164 is also fed to an analog to digital convertor 170, which is in turn coupled to a microprocessor-based microcomputer 172.

In order to automatically determine the pH of the wall of the hollow viscous organ under test, a separate gas analyzer sensor 174 is used to determine the bicarbonate concentration in the arterial blood of the patient. The output of sensor 174 is coupled through analog to digital convertor 176 to microcomputer 172. Microcomputer 172 is preprogrammed to calculate the pH of the organ wall using the values provided by analog to digital convertors 170 and 176. Conversion of pCO₂ measurements can be converted into pH measurements automatically by microcomputer 172 using various equations and references well-known in the art.

Although many different types of output devices may be employed, strip chart recorder 178 and CRT monitor 180 have been illustrated. Strip chart recorder 178 and monitor 180 are coupled as output devices to microcomputer 172. Strip chart recorder 178 offers the advantage of developing an easily readable, permanent record of the fluctuations in organ wall pH. Monitor 180 offers the advantage of providing digital readout of the pH value as well as displaying the upper and lower excursions of pH fluctuation. If desired, microcomputer 172 can be preprogrammed using keyboard 182 to compare the instantaneous pH value with doctor-selected upper and lower alarm limits. If the measured instantaneous pH fluctuates outside those limits, microcomputer 172 can sound an alarm to alert hospital staff.

While a single semiconductor device 46 has been illustrated in conjunction with the electronic circuit of Figure 9, the circuit may be readily adapted for use with a plurality of semiconductor devices in order to measure the pH at different locations substantially simultaneously. In such an embodiment, the data coming from each sensor can be fed to a separate I/O port of microcomputer 172. In the alternative, a single I/O port can be used with the individual input signals being time multiplexed.

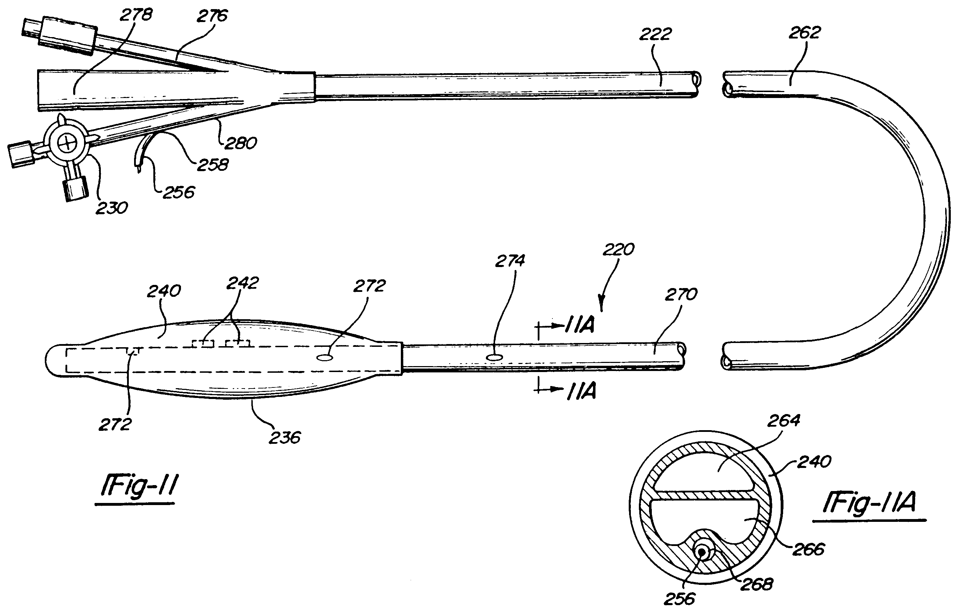

As an alternative to electronic pH sensors, the invention may also be practiced using optical sensor technology. Referring to Figure 10, the presently preferred optical sensor embodiment uses a first fiber optic cable 94 which is optically coupled through a series of lenses 96, selectable color filters 98 and heat absorber 100 to an illumination source 102, such as a 100 watt tungsten-halogen lamp. Fiber optic cable 94 is routed through the tonometric catheter lumen in a fashion similar to the conductor 56 of the above-described embodiments, with the end thereof protruding through the tubing and into the sampling chamber 40. A second fiber optic cable 104 is routed parallel to the first fiber optic cable 94, with one end protruding through the tubing and held in place adjacent the end of first cable 94 with a collar 106. Collar 106 may be adhesively bonded to the outside wall of the tubing. The opposite end of second fiber optic cable 104 is positioned for optically coupling with a phototransistor 108 which is electrically connected to an operational amplifier circuit 110. The operational amplifier circuit can be coupled to an analog to digital converter, such as A/D converter 170 of Figure 7.

In use, fiber optic cable 94 illuminates a region within the sampling chamber 40 which is filled with a sampling fluid containing a colorimetric pH indicator. The illumination from fiber optic cable 94 reflects from the molecules suspended in the pH indicator solution, with some of the reflected illumination passing back through second fiber optic cable 104 to the phototransistor. By selecting the appropriate filter 98, a monochromatic illumination or illumination of otherwise known spectral content is employed to illuminate the colorimetric pH indicator solution. When the color of the filtered illumination matches that of the indicator, the illumination is absorbed and a low illumination signal is received at the phototransistor. When a pH change causes a color change in the indicator away from the color of the filtered illumination, more illumination is reflected back to the phototransistor, with an attendant increase in detected signal output. In this fashion, the proper selection of indicator dye and illumination filtration can be used to detect pH ranges. For a further description of fiber optic pH sensor technology, refer to G. G. Vurek "A Fiber Optic pCO₂ Sensor," Annals of Biomedical Engineering, Vol. 11, pp. 499-510, 1983, which is available from Pergamon Press, Ltd., and is expressly incorporated herein by reference.

While the preferred embodiments have been disclosed in connection with monitoring of the gastrointestinal tract and the urinary and ureteric tracts it will be appreciated that its principles are applicable to other hollow internal organs to monitor pH and hence perfusion of those organs. Also while several presently preferred detailed constructions for tonometric catheters have been disclosed, it will be appreciated that other constructions may be developed which are equally suitable. The disclosed constructions are presently preferred for the reason that they are readily fabricated using existing available materials. Other embodiments may include other, but equivalent materials for the tonometric catheter membrane and/or connective tubing. They may also differ in the specific fabrication details. As an example, the sampling chamber may be eccentric rather than symmetric about the connective tubing.

Another embodiment of the tonometric catheter is illustrated in Figures 11 and 11A. As illustrated, the tonometric catheter is appropriately configured to also serve as a urinary or ureteric catheter, either with or without suction, which optionally employs sensors. With reference to Figures 11 and 11A, the tonometric catheter 220 comprises a multipassage tubing 262 which defines three individual noncommunicating (between each other) passageways or lumens, an optional air or irrigation lumen 264, a drainage or suction lumen 266 and a tonometric catheter lumen 268. A tonometric catheter membrane, similar to that previously described, is attached at a distal location on tubing 262, allowing an intermediate portion of the tubing not extending beyond the end of membrane 236 to define the uretary or uretary catheter 270. Tubing 262 is provided with a plurality of perforations 272 which communicate between tonometric catheter lumen 268 and the sampling chamber 240 defined by membrane 236. If desired, one or more sensors 242 can be included in accordance with the above teachings, in which case a suitable conductor 256 may be routed through tonometric catheter lumen 268 to exit at sealed aperture 258.

The urinary catheter or ureteric catheter portion 270 is suitably provided with a plurality of openings 274 through which the bladder or ureters may be aspirated or irrigated.

At the opposite end of tubing 262 the tubing splits to form three separate connections. Air or irrigation lumen 264 optionally communicates with air lumen passageway 276, urinary lumen connects with suction or drainage lumen passageway 278 and tonometric catheter lumen 268 communicates with tonometric catheter lumen passageway 280. The tonometric catheter lumen passageway is fitted with three-way stopcock 230, similar in function and purpose to the three-way stopcock 30 described in connection with Figure 1. If desired, a quick connect fitting 82 as seen in Figure 4 may be used to couple the suction urinary passageway 278 with an aspiration source. As illustrated, the quick connect fitting preferably has angularly cut ends and a slightly enlarged midsection, making it easy to insert into the end of passageway 278 and also into the aspiration hose coupling (not shown). The enlarged midsection helps form a seal with the adjoining passageways. Preferably the quick connect fitting is fabricated of disposable plastic.

Yet another embodiment of the urinary catheter/tonometric catheter combination illustrated in Figures 11 and 11A may employ a multiple tonometric catheter embodiment employing a tubing having a plurality of passageways or lumen as shown in the cross-sectional view of Figure 5A.

In another embodiment of the present invention, a tonometric catheter may be adopted to deliver a pharmaceutically-active agent, either for systemic, local or topical activity, or a combination thereof. For example, an additional lumen may be added such as that and for irrigation or aspiration, to deliver the active. For example, the irrigation/aspiration lumen 264 shown in Figure 11 and 11A, may be used to deliver an active agent. In another embodiment, a portion of the device may be modified so as to provide sustained release of the active agent of interest.

Thus, for example, the problems of nosacomial infection associated with catheter insertion can be overcome by incorporating an antimicrobial into at least a portion of the polymeric material used to manufacture the tonometric catheter, or by coating at least a portion of the device with a sustained release composition, or by delivering the antimicrobial via the tonometric catheter. Such modifications are well known to those skilled in the art. See U.S. Patent No. 4,677,143, incorporated herein by reference.

Classes of useful agents include antimicrobial agents, nonsteroidal anti-inflammatory agents, topical anesthetics, topical vasodialators, metabolic suppressants, and other agents that could be delivered for absorption at the sites of the tonometric catheter.

Accordingly, while several preferred embodiments of the invention have been disclosed, it will be appreciated that principles of the invention, as set forth in the following claims, are applicable to other embodiments.

## Claims

1. A tonometric catheter apparatus (20) for remotely determining a fluid or gas property indicative of the condition of a hollow internal organ of a human or other mammal in vivo as defined by said fluid or gas property comprising:
(a) an elongated tonometric catheter tube (22) having a lumen (68) extending longitudinally therethrough, said tube (22) being composed of a first elastomeric material that is substantially impermeable to one or more fluids or gases of interest, said fluids of interest including oxygen gases and carbon dioxide gases;
(b) at least one walled sampling chamber (40) on said catheter tube (22) in fluid communication with the interior of said lumen (68), said walled sampling chamber (40) being defined by a balloon member generally surrounding a portion of said tube (22) and sealingly interconnected therewith, said balloon member being adapted to form an interior space between said balloon member and said catheter tube (22), the wall (36) of said balloon member being composed of a second material that is freely and selectively permeable to said one or more fluids or gases of interest, said second material being substantially impermeable to other fluids or gases, with said tube (22) extending to a position outside of the body of the human or other mammal, and with said lumen (68) providing fluid communication between said sampling chamber and the outside of the body of the human or other mammal;
(c) sensor means (42) for sensing the level of at least one of said fluids or gases of interest present within said sampling chamber (40);
characterised in that:
said sensor means (42) is disposed within said sampling chamber (40);
said apparatus further comprises calibration means for introducing a calibration fluid into said sampling chamber through said lumen in order to calibrate or recalibrate said sensor means; and
a portion of said balloon member may be selectively positioned substantially adjacent to a wall portion of the internal organ in order to allow said one or more fluids or gases of interest from the tissue of the wall portion of the internal organ to permeate into said sampling chamber (40), the sensor means (42) being adapted to sense the level of at least one of the fluids or gases of interest from the tissue of the wall portion of the internal organ, and the calibration means being operable whilst said balloon member is substantially adjacent to the wall portion of the internal organ.

2. An apparatus (20) according to claim 1 for determining the pH of a hollow internal organ of a human or other mammal, characterised in that:
said tube (22) is composed of a first elastomeric material that is substantially impermeable to a fluid or gas indicative of pH;
said wall (36) of said balloon member is composed of a second material that is freely and selectively permeable to said fluid or gas indicative of pH;
a non-temperature sensor (42) is disposed on said tonometric catheter tube (22) in communication with said sampling chamber (40) for introduction into said organ with said tonometric catheter, said sensor (42) comprising means responsive to a fluid or gas property indicative of pH and means for developing an electromagnetic signal indicative of said fluid or gas property;
a means is provided responsive to said signal for generating a pH signal indicative of the pH of said organ; and
said position of said balloon member substantially adjacent to said wall portion of said hollow internal organ is such to allow said fluid or gas indicative of pH from the tissue of the wall portion of the internal organ to permeate into said sampling chamber (40).

3. An apparatus (20) according to claim 1 for determining the condition of a hollow internal organ of a human or other mammal as defined by the fluid pressure and the blood pH indicative of the condition of the hollow internal organ, characterised in that:
said tube (22) is composed of a first elastomeric material that is substantially impermeable to a fluid or gas indicative of the fluid pressure of the hollow internal organ;
said wall (36) of said balloon member is composed of a second material that is freely and selectively permeable to said fluid or gas indicative of the fluid pressure of the hollow internal organ;
a non-temperature sensor (42) is disposed on said tonometric catheter tube (22) in communication with said sampling chamber (40) for developing a first electromagnetic signal indicative of said fluid pressure condition of said hollow internal organ;
a means (174) is provided for developing a second electromagnetic signal indicative of said blood pH of said hollow internal organ;
a means (170,176,172) is provided receptive of said first and second signals for generating a third signal indicative of the condition of said hollow internal organ;
a means (178) is provided responsive to said third signal and located outside said organ for reporting said condition of said hollow internal organ; and
said position of said balloon member substantially adjacent to a wall portion of said hollow internal organ is such to allow said fluid or gas indicative of the fluid pressure from the tissue of the wall portion of the internal organ to permeate into said sampling chamber (40).

4. An apparatus according to claim 1 or claim 2 or claim 3, characterised in that it additionally comprises a temperature sensor in communication with said sampling chamber (40).

5. An apparatus according to claim 1 or claim 2 or claim 3, characterised in that said sensor (42) comprises a chemically sensitive electronic transducer.

6. An apparatus according to claim 1 or claim 2 or claim 3, characterised in that said sensor (42) comprises a chemically sensitive field effect transistor transducer.

7. An apparatus according to claim 2, characterised in that said electromagnetic signal is an electrical signal conveyed by wire (56) to said means for generating a pH signal.

8. An apparatus according to claim 3, characterised in that said first electromagnetic signal is an electrical signal conveyed by wire (56) to said means (172) for generating said third signal.

9. An apparatus according to claim 3, characterised in that said means (178) for reporting said condition includes analog circuit means for producing a report of said condition.

10. An apparatus according to claim 3, characterised in that said means (178) for reporting said condition includes digital circuit means for producing a report of said condition.

11. An apparatus according to claim 3, characterised in that said tonometric catheter (20) additionally comprises a second electronic sensor for determining the temperature of the sampling area adjacent said first sensor.

12. An apparatus according to claim 3, characterised in that the fluid or gas property of interest is pCO₂.

13. An apparatus according to claim 3, characterised in that the fluid or gas property of interest is pO₂.

14. An apparatus according to any preceding claim characterised in that at least a portion of the tonometric catheter (20) comprises α-hydro-Ω-hydroxypoly-(oxytetramethylene).

## Patentansprüche

1. Eine tonometrische Kathetervorrichtung (20) zur Fernermittlung einer Eigenschaft eines Fluides oder Gases, wobei diese Eigenschaft für den Zustand eines innerlichen Hohlorgans in vivo eines Menschen oder eines anderen Säugetieres kennzeichnend ist, wie durch die genannte Eigenschaft des Fluids oder Gases definiert, aufweisend:
(a) eine langgestreckte tonometrische Katheterleitung (22) mit einem Lumen (68), das sich in Längsrichtung durch sie hindurch erstreckt, wobei die Leitung (22) aus einem ersten elastomeren Material gebildet ist, das im wesentlichen für ein oder mehrere Fluide oder Gase von Interesse undurchlässig ist, und wobei die genannten Fluide von Interesse Sauerstoffgase und Kohlendioxidgase mit einschließen;
(b) wenigstens eine durch eine Wand umgebene Probenkammer (40) an der Katheterleitung (22) in Fluidverbindung mit dem Inneren des genannten Lumens (68), wobei die genannte, durch eine Wand umgebene Probenkammer (40) durch ein Ballonelement gebildet ist, das im allgemeinen einen Bereich der genannten Leitung (22) umgibt und mit dieser in abgedichteter Weise verbunden ist, wobei das genannte Ballonelement dazu befähigt ist, einen inneren Raum zwischen dem Ballonelement und der Katheterleitung (22) zu bilden, wobei die Wand (36) des genannten Ballonelements aus einem zweiten Material gebildet ist, das für eine oder mehrere Fluide oder Gase von Interesse frei und selektiv durchlässig ist, wobei das genannte zweite Material im wesentlichen für andere Fluide oder Gase undurchlässig ist, wobei die genannte Leitung (22) sich bis zu einer Position außerhalb des Körpers des Menschen oder des anderen Säugetieres erstreckt und wobei das genannte Lumen (68) eine Fluidverbindung zwischen der genannten Probenkammer und der Außenseite des Körpers des Menschen oder des anderen Säugetieres ergibt;
(c) eine Sensoreinrichtung (42) zum Abfühlen des Spiegels wenigstens eines der innerhalb der Probenkammer (40) vorhandenen Fluide oder Gase von Interesse;
**dadurch gekennzeichnet**, daß:
die genannte Sensoreinrichtung (42) innerhalb der genannten Probenkammer (40) angeordnet ist;
die genannte Vorrichtung ferner eine Kalibriereinrichtung zum Einleiten eines Kalibrierfluids in die genannte Probenkammer durch das genannte Lumen aufweist, um die genannte Sensoreinrichtung zu kalibrieren oder nachzukalibrieren; und
ein Bereich des genannten Ballonelements selektiv im wesentlichen benachbart zu einem Wandbereich des innerlichen Organs positioniert werden kann, um es einem oder mehreren Fluiden oder Gasen von Interesse zu erlauben, von dem Gewebe des Wandbereichs des innerlichen Organs in die genannte Probenkammer (40) einzudringen, wobei die Sensoreinrichtung (42) dazu befähigt ist, den Spiegel von wenigstens einem der Fluide oder Gase von Interesse von dem Gewebe des Wandbereiches des innerlichen Organes abzufühlen, und wobei die Kalibriereinrichtung betriebsfähig ist, während das genannte Ballonelement im wesentlichen dem Wandbereich des innerlichen Organs benachbart ist.

2. Eine Vorrichtung (20) gemäß Anspruch 1 zum Bestimmen des pH eines innerlichen Hohlorgans eines Menschen oder eines anderen Säugetieres,
**dadurch gekennzeichnet**, daß:
die genannte Leitung (22) aus einem ersten elastomeren Material gebildet ist, das für ein für den pH kennzeichnendes Fluid oder Gas im wesentlichen undurchlässig ist;
die genannte Wand (36) des genannten Ballonelements aus einem zweiten Material besteht, das für das für den pH kennzeichnende Fluid oder Gas frei und selektiv durchlässig ist;
ein Nichttemperatur-Sensor (42) an der tonometrischen Katheterleitung (22) in Verbindung mit der Probenkammer zum Einführen in das Organ mit dem tonometrischen Katheter angeordnet ist, wobei der Sensor (42) aufweist: Mittel, die auf eine Eigenschaft eines Fluides oder Gases ansprechen, wobei diese Eigenschaft für den pH kennzeichnend ist, sowie Mittel zum Erzeugen eines elektromagnetischen Signals, das für die Eigenschaft des genannten Fluids oder Gases kennzeichnend ist;
eine Einrichtung vorgesehen ist, die auf das Signal anspricht, um ein pH-Signal zu erzeugen, das für den pH des genannten Organs kennzeichnend ist; und
die Position des Ballonelements, das im wesentlichen dem Wandbereich des innerlichen Hohlorganes benachbart ist, derart ist, daß es dem für den pH kennzeichnenden Fluid oder Gas ermöglicht wird, von dem Gewebe des Wandbereiches des innerlichen Organs in die Probenkammer (40) zu dringen.

3. Eine Vorrichtung (20) gemäß Anspruch 1, zum Bestimmen des Zustandes eines innerlichen Hohlorganes eines Menschen oder eines anderen Säugetieres, wobei dieser Zustand durch den Fluiddruck und den Blut-pH-Wert definiert ist, die für den Zustand des innerlichen Hohlorgans kennzeichnend sind,
**dadurch gekennzeichnet**, daß:
die Leitung (22) aus einem ersten elastomeren Material gebildet ist, das für ein für den Fluiddruck des innerlichen Hohlorgans kennzeichnendes Fluid oder Gas im wesentlichen undurchlässig ist;
die Wand (36) des Ballonelementes aus einem zweiten Material gebildet ist, das für das für den Fluid-Druck des innerlichen Hohlorganes kennzeichnende Fluid oder Gas frei und selektiv durchlässig ist;
ein Nichttemperatur-Sensor (42) an der tonometrischen Katheterleitung (22) in Verbindung mit der Probenkammer (40) zum Erzeugen eines ersten elektromagnetischen Signals angeordnet ist, das für den Fluiddruck-Zustand des innerlichen Hohlorgans kennzeichnend ist;
eine Einrichtung (174) vorgesehen ist, um ein zweites elektromagnetisches Signal zu erzeugen, das für den Blut-pH-Wert des innerlichen Hohlorgans kennzeichnend ist;
eine Einrichtung (170, 176, 172) vorgesehen ist, welche für das erste Signal und das zweite Signal aufnahmefähig ist, um ein drittes Signal zu erzeugen, das für den Zustand des innerlichen Hohlorgans kennzeichnend ist;
eine Einrichtung (178) vorgesehen ist, die auf das dritte Signal anspricht und außerhalb des Organs zum Melden des Zustandes des innerlichen Hohlorgans angeordnet ist; und
die Position des Ballonelementes, das im wesentlichen einem Wandbereich des innerlichen Hohlorgans benachbart ist, derart ist, daß es dein für den Fluid-Druck kennzeichnenden Fluid oder Gas ermöglicht wird, von dem Gewebe des Wandbereiches des innerlichen Organs in die Probenkammer (40) zu dringen.

4. Eine Vorrichtung gemäß Anspruch 1 oder Anspruch 2 und Anspruch 3,
**dadurch gekennzeichnet**,
daß sie zusätzlich einen Temperatursensor in Verbindung mit der Probenkammer (40) aufweist.

5. Eine Vorrichtung gemäß Anspruch 1 oder Anspruch 2 oder Anspruch 3,
**dadurch gekennzeichnet**,
daß der Sensor (42) einen chemisch empfindlichen elektronischen Meßwandler aufweist.

6. Eine Vorrichtung gemäß Anspruch 1 oder Anspruch 2 oder Anspruch 3,
**dadurch gekennzeichnet**,
daß der Sensor (42) einen chemisch empfindlichen Meßwandler mit Feldeffekttransistor aufweist.

7. Eine Vorrichtung gemäß Anspruch 2,
**dadurch gekennzeichnet**,
daß das elektromagnetische Signal ein elektrisches Signal ist, das durch Draht (56) zu der Einrichtung zum Erzeugen eines pH-Signales geleitet wird.

8. Eine Vorrichtung gemäß Anspruch 3,
**dadurch gekennzeichnet**,
daß das erste elektromagnetische Signal ein elektrisches Signal ist, das durch Draht (56) zu der Einrichtung (172) zum Erzeugen des dritten Signales geleitet wird.

9. Eine Vorrichtung gemäß Anspruch 3,
**dadurch gekennzeichnet**,
daß die Einrichtung (178) zum Melden des Zustandes eine Analogschaltung zum Erzeugen einer Meldung über den Zustand aufweist.

10. Eine Vorrichtung gemäß Anspruch 3,
**dadurch gekennzeichnet**,
daß die Einrichtung (178) zum Melden des Zustandes eine Digitalschaltung zum Erzeugen einer Meldung über den Zustand aufweist.

11. Eine Vorrichtung gemäß Anspruch 3
**dadurch gekennzeichnet**,
daß der tonometrische Katheter (20) zusätzlich einen zweiten elektronischen Sensor zum Ermitteln der Temperatur des Probenbereiches in Nachbarschaft zu dem ersten Sensor aufweist.

12. Eine Vorrichtung gemäß Anspruch 3,
**dadurch gekennzeichnet**,
daß die Fluid- oder Gas-Eigenschaft von Interesse pCO₂ ist.

13. Eine Vorrichtung gemäß Anspruch 3,
**dadurch gekennzeichnet**,
daß die Fluid- oder Gas-Eigenschaft von Interesse pO₂ ist.

14. Eine Vorrichtung gemäß einem der vorhergehenden Ansprüche,
**dadurch gekennzeichnet**,
daß zumindest ein Bereich des tonometrischen Katheters (20) α-hydro-Ω-hydroxypoly-(oxytetramethylen) aufweist.

## Revendications

1. Appareil cathéter tonométrique (20) pour déterminer à distance in vivo une propriété d'un fluide ou d'un gaz indicative de la condition d'un organe interne creux d'une personne humaine ou d'un autre mammifère, telle que définie par ladite propriété du fluide ou du gaz, comprenant :
(a) un tube cathéter tonométrique allongé (22) ayant un passage (68) s'étendant longitudinal à travers lui, ledit tube (22) étant en un premier matériau élastomère pratiquement imperméable à un ou plusieurs fluides ou gaz intéressants, ces fluides intéressants comprenant l'oxygène gazeux et le dioxyde de carbone gazeux;
(b) au moins une chambre d'échantillonnage close (40) sur ledit tube cathéter (22) en communication fluide avec l'intérieur dudit passage (68), ladite chambre d'échantillonnage close (40) étant définie par un élément de ballon entourant généralement une portion dudit tube (22) et fixée de façon étanche sur lui, ledit élément de ballon étant adapté pour former un espace intérieur entre ledit élément de ballon et ledit tube cathéter (22), la paroi (36) dudit élément de ballon étant en un deuxième matériau qui est librement et sélectivement perméable à ce(s) fluide(s) ou gaz intéressant(s), ledit deuxième matériau étant pratiquement imperméable à d'autres fluides ou gaz, ledit tube (22) s'étendant jusqu'à une position à l'extérieur du corps de la personne humaine ou d'un autre mammifère, et ledit passage (68) procurant une communication fluide entre ladite chambre d'échantillonnage et l'extérieur du corps de la personne humaine ou de l'autre mammifère;
(c) des moyens de capteur (42) pour détecter le niveau d'au moins l'un desdits fluides ou gaz intéressants présents à l'intérieur de ladite chambre d'échantillonnage (40);
caractérisé en ce que :
lesdits moyens de capteur (42) sont disposés à l'intérieur de ladite chambre d'échantillonnage (40);
ledit appareil comprend en outre des moyens d'étalonnage pour introduire un fluide d'étalonnage dans ladite chambre d'échantillonnage à travers ledit passage afin d'étalonner ou de réétalonner lesdits moyens de capteur; et
une portion dudit élément de ballon peut être sélectivement positionnée pratiquement adjacente à une portion de paroi de l'organe interne afin de permettre à ce(s) fluide(s) ou gaz intéressant(s) en provenance du tissu de la portion de paroi de l'organe interne de passer dans ladite chambre d'échantillonnage (40), les moyens de capteur (42) étant adaptés pour détecter le niveau d'au moins l'un des fluides ou gaz intéressants en provenance du tissu de la portion de paroi de l'organe interne, et les moyens d'étalonnage pouvant être mis en oeuvre pendant que ledit élément de ballon est pratiquement adjacent à la portion de paroi de l'organe interne.

2. Appareil (20) selon la revendication 1, pour déterminer le pH d'un organe interne creux d'une personne humaine ou d'un autre mammifère, caractérisé en ce que :
ledit tube (22) est un premier matériau élastomère pratiquement imperméable à un fluide ou un gaz indicatif de pH;
ladite paroi (36) dudit élément de ballon est en un deuxième matériau qui est librement et sélectivement perméable audit fluide ou gaz indicatif de pH;
un capteur non sensible à la température (42) est disposé sur ledit tube cathéter tonométrique (22) en communication avec ladite chambre d'échantillonnage (40) pour introduction dans ledit organe avec ledit cathéter tonométrique, ledit capteur (42) comprenant des moyens sensibles à une propriété d'un fluide ou d'un gaz indicative de pH et des moyens pour développer un signal électromagnétique indicatif de ladite propriété du fluide ou du gaz;
il est prévu un moyen sensible audit signal pour produire un signal de pH indicatif du pH dudit organe; et
ladite position dudit élément de ballon pratiquement adjacente à ladite portion de paroi dudit organe interne est telle qu'elle permet audit fluide ou gaz indicatif de pH en provenance du tissu de la portion de paroi de l'organe interne de passer dans ladite chambre d'échantillonnage (40).

3. Appareil (20) selon la revendication 1 pour déterminer la condition d'un organe interne creux d'une personne humaine ou d'un autre mammifère telle que définie par la pression de fluide et le pH du sang indicatifs de la condition de l'organe interne creux, caractérisé en ce que :
ledit tube (22) est en un premier matériau élastomère pratiquement imperméable à un fluide ou gaz indicatif de la pression de fluide de l'organe interne creux;
ladite paroi (36) dudit élément de ballon est en un deuxième matériau qui est librement et sélectivement perméable audit fluide ou gaz indicatif de la pression de fluide de l'organe interne creux;
un capteur non sensible à la température (42) est disposé sur ledit tube cathéter tonométrique (22) en communication avec ladite chambre d'échantillonnage (40) pour développer un premier signal électromagnétique indicatif de ladite condition de pression de fluide dudit organe interne creux;
il est prévu un moyen (174) pour développer un deuxième signal électromagnétique indicatif dudit pH du sang dudit organe interne creux;
il est prévu un moyen (170, 176, 172) pour recevoir lesdits premier et deuxième signaux pour produire un troisième signal indicatif de la condition dudit organe interne creux;
il est prévu un moyen (178) sensible audit troisième signal et disposé à l'extérieur dudit organe pour rapporter ladite condition dudit organe interne creux; et
ladite position dudit élément de ballon pratiquement adjacente à une portion de paroi dudit organe interne creux est telle qu'elle permet audit fluide ou gaz indicatif de la pression de fluide en provenance du tissu de la portion de paroi de l'organe interne de passer dans ladite chambre d'échantillonnage (40).

4. Appareil selon la revendication 1 ou la revendication 2 ou la revendication 3, caractérisé en ce qu'il comprend en outre un capteur de température en communication avec ladite chambre d'échantillonnage (40).

5. Appareil selon la revendication 1 ou la revendication 2 ou la revendication 3, caractérisé en ce que ledit capteur (42) comprend un transducteur électronique chimiquement sensible.

6. Appareil selon la revendication 1 ou la revendication 2 ou la revendication 3, caractérisé en ce que ledit capteur (42) comprend un transducteur à transistor à effet de champ chimiquement sensible.

7. Appareil selon la revendication 2, caractérisé en ce que ledit signal électromagnétique est un signal électrique transporté par un fil métallique (56) auxdits moyens de production d'un signal de pH.

8. Appareil selon la revendication 3, caractérisé en ce que ledit premier signal électromagnétique est un signal électrique transporté par un fil métallique (56) auxdits moyens (172) de production dudit troisième signal.

9. Appareil selon la revendication 3, caractérisé en ce que lesdits moyens (178) pour rapporter ladite condition comprennent un circuit analogique pour produire un compte rendu de ladite condition.

10. Appareil selon la revendication 3, caractérisé en ce que lesdits moyens (178) pour rapporter ladite condition comportent un circuit numérique pour produire un compte rendu de ladite condition.

11. Appareil selon la revendication 3, caractérisé en ce que ledit cathéter tonométrique (20) comprend en outre un deuxième capteur électronique pour déterminer la température de la zone d'échantillonnage adjacente audit premier capteur.

12. Appareil selon la revendication 3, caractérisé en ce que la propriété du fluide ou du gaz intéressante est pCO₂.

13. Appareil selon la revendication 3, caractérisé en ce que la propriété du fluide ou du gaz intéressante est pO₂.

14. Appareil selon l'une des revendications précédentes, caractérisé en ce qu'au moins une portion du cathéter tonométrique (20) comprend α-hydro-Ω-hydroxypoly-(oxytétraméthylène).
